# EUROPEAN PATENT APPLICATION

(11) **EP 2 564 796 A1**
(43) Date of publication of application: **06.03.2013**
(21) Application number: 11844959.4
(22) Date of filing: 02.12.2011
(51) Int. Cl.: A61B 17/3211, A61B 19/00

(54) **MEDICAL INSTRUMENT**

(30) Priority: 03.12.2010 JP 2010270579
(71) Applicant: Nakamura, Shoichi, Higashichikuma-gun Nagano 399-7502 (JP); ACP Japan Co. Ltd., Tokyo 113-0033 (JP)
(72) Inventor: NAKAMURA, Soichi, ^Nagano 399-7502 (JP)
(74) Representative: Gassner, Wolfgang
(86) International application number: PCT/JP2011/077889
(87) International publication number: WO 2012/074084

(57) **Abstract**

To provide a medical instrument for enabling a functional portion provided at the front end of the instrument to be attached and detached to be disposable, while enabling the angle to be adjusted freely to ensure excellent visual recognition, in a medical instrument 10A provided with a rod-shaped grasp portion 20 at its base end and a functional portion 40A used in a medical practice at its front end, a shape memory portion 30 made of a rod-shaped shape memory alloy is provided in between the grasp portion 20 and the functional portion 40A, the grasp portion 20, the functional portion 40A and the shape memory portion 30 are made mutually attachable and detachable, and the shape memory portion 30 is subjected to memory treatment in a linear state, is bent as necessary to use, and is returned to its original shape subjected to the memory treatment by heating the shape memory portion 30 to a memory recovery temperature after using.

## Description

### Technical Field

The present invention relates to medical instruments such as a knife, mirror and hook used in the medical field, and more particularly, to a medical instrument for enabling a functional portion provided at the front end of the instrument to be attached and detached to be replaceable.

### Background Art

In a medical instrument provided with a rod-shaped grasp portion at its base end and a functional portion such as a knife, mirror and hook used in a medical practice at its front end, it has conventionally been general that the grasp portion and the functional portion are arranged substantially linearly.

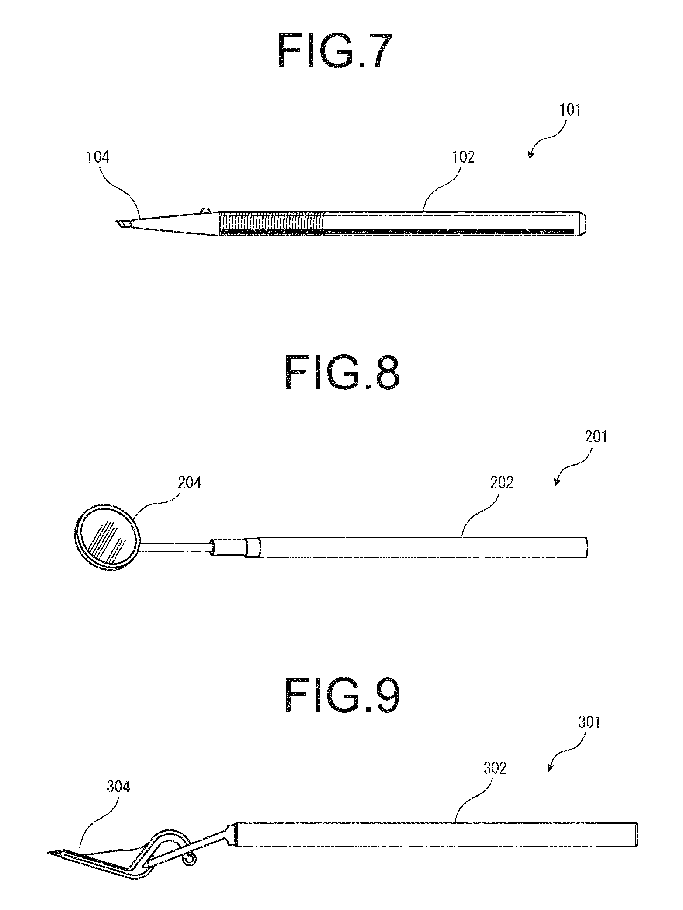

FIGs. 7 and 8 show examples of such conventional medical instruments. FIG. 7 is a schematic view showing an example of the conventional medical knife, and FIG. 8 is a schematic view showing an example of the conventional medical mirror.

The medical knife 101 as shown in FIG. 7 has a rod-shaped grasp portion 102 at its base end, while being provided at its front end with a knife body 104 as a functional portion. Meanwhile, the medical mirror 201 as shown in FIG. 8 has a rod-shaped grasp portion 202 at its base end, while being provided at its front end with a mirror body 204 as a functional portion. As shown in the figures, the grasp portions 102, 202 and functional portions 104, 204 are arranged approximately in a straight line fixedly, respectively.

However, for example, in surgery of coronary artery bypass of the heart and the like, from the relationship between a position of a blood vessel to incise and an insertion angle of a medical knife, in the conventional medical knife as shown in FIG. 7, the knife front end is sometimes hidden behind the hand of the operator. Therefore, in the medical knife 101 that the grasp portion 102 and knife body 104 are arranged in a straight line as described above, there is a problem that the knife is hard to see in incision and difficult to use.

Then, medical knives such that a detachable knife body can be arranged at an arbitrary angle with respect to the grasp portion have been commercially available previously (for example, Patent Document 1). FIG. 9 shows a conventional medical knife. The medical knife 301 is comprised of a grasp portion 302 and a knife body (functional portion) 304 in the shape of a "C". The knife body 304 is bent in the shape of a "C" while a connection portion between the grasp portion 302 and the knife body 304 is bent, and therefore, it is possible to attach a blade body to the grasp portion 302 at a certain angle. As the knife body 304, several kinds with different bend angles are prepared, and knife bodies 304 of arbitrary angles are replaced and used as necessary.

Meanwhile, the medical mirror 201 is used for visual recognition of a site hard to see in general such as a deep narrow operation site and inside the mouse, and conventionally, makes an observation target easy to see by attaching the mirror body 204 to the grasp portion 202 at an angle as shown in FIG. 8.

### Prior Art Document

### Patent Document

Patent Document 1: Japanese Patent Gazette No. 3980989

### Disclosure of Invention

### Problems to be Solved by the Invention

In the case of the above-mentioned conventional medical knife 301 as shown in FIG. 9, since the bend angles of the knife body 304 are beforehand set, there is a problem that the angle of the knife body 304 can be changed only stepwise. In other words, flexibility in the change angle of the knife body 304 is low, and it is not possible to set a delicate angle corresponding to the case. Further, in the case of changing to a different angle during treatment, there is a problem that a new medical knife 301 is prepared again or the knife body 304 is removed from the grasp portion 302 to be replaced with the knife body 304 of a different bend angle.

Meanwhile, in the case of the above-mentioned conventional medical mirror 201, since the mirror body 204 is only provided with an angle, visual recognition is poor depending on the site, and further, since the angle of the mirror 204 is fixed, there is a problem that it is not possible to change the angle as necessary.

Further, in the case that the grasp portions 102, 202 and functional portions 104, 204 are integrally formed as shown in the above-mentioned conventional medical instruments 101, 201 as shown in FIGs. 7 and 8, respectively, the instrument is cleaned and sterilized every time after using to be used repeatedly, or the only alternative is to make the whole disposable. However, in the case of sterilizing to reuse, there is a possibility of contacting the functional portion to which bacteria and the like adhere after being used and acquiring an infection, and there is a risk of receiving a wound in the hand, figure and the like by the blade or the like. Meanwhile, when the whole is made disposable, the need of cleaning and the like is eliminated, but from the relationship with the cost, the grasp portions 102, 202 are formed from an inexpensive light material such as plastic instead of a titanium alloy, stainless steel and the like. However, when the grasp portions 102, 202 are light, the knife is not stabilized at hand in using, and there is a difficulty that the knife is hard to use in precision work.

Therefore, in view of the aforementioned circumstances, it is an object of the present invention to provide a medical instrument for enabling a functional portion provided at the front end of the instrument to be attached and detached to be disposable, while enabling the angle to be adjusted freely to ensure excellent visual recognition.

### Means for Solving the Problem

To attain the aforementioned object, the present invention provides a medical instrument provided with a rod-shaped grasp portion at its base end and a functional portion used in a medical practice at its front end characterized by having a shape memory portion made of a rod-shaped shape memory alloy between the grasp portion and the functional portion, where the grasp portion, the functional portion and the shape memory portion are made mutually attachable and detachable, and the shape memory portion is subjected to memory treatment in a linear state, while being bent as necessary to use, and returns to its original shape subjected to the memory treatment by heating the shape memory portion to a memory recovery temperature after using.

Herein, the medical instrument may be further provided with a connection portion bent at a predetermined angle between the grasp portion and the shape memory portion or between the shape memory portion and the functional portion.

The shape memory portion is preferably made of a nickel-titanium alloy. Further, the medical instrument is used in a medical knife, a medical mirror, a medical hook and the like.

In addition, as the functional portion, a plurality of shape types is enclosed in individual storage means, and any one of the plurality of shape types is selected and installed in an installation portion of the shape memory portion to be used. Further, the functional portion enclosed in the storage means is sterilized and stored.

### Advantageous Effect of the Invention

According to the medical instrument of the invention, by providing the shape memory portion, the angle of the functional portion is made adjustable freely, and it is possible to ensure excellent visual recognition. Further, each portion is made attachable/detachable, only the functional portion is made disposable, and it is thereby possible to suppress the cost while considering safety and sanitation. Brief Description of Drawings

FIG. 1 is an entire front view showing the outline of a medical instrument (medical knife) according to Embodiment 1 of the invention;
FIG. 2 is an entire front view showing a state in which a shape memory portion of the medical instrument as shown in FIG. 1 is bent;
FIG. 3 contains schematic views showing examples of a shape of a blade body of the medical knife;
FIG. 4 contains entire front views showing the outline of the medical instrument (medical mirror) according to Embodiment 1 of the invention;
FIG. 5 contains entire front views showing the outline of the medical instrument (medical hook) according to Embodiment 1 of the invention;
FIG. 6 contains entire front views showing the outline of a medical instrument (medical knife) according to Embodiment 2 of the invention;
FIG. 7 is a schematic view (part 1) showing an example of a conventional medical knife;
FIG. 8 is a schematic view showing an example of a conventional medical mirror; and
FIG. 9 is a schematic view (part 2) showing another example of the conventional medical knife.

### Mode for Carrying Out the Invention

Embodiments of the invention will specifically be described below with reference to drawings.

### [Embodiment 1]

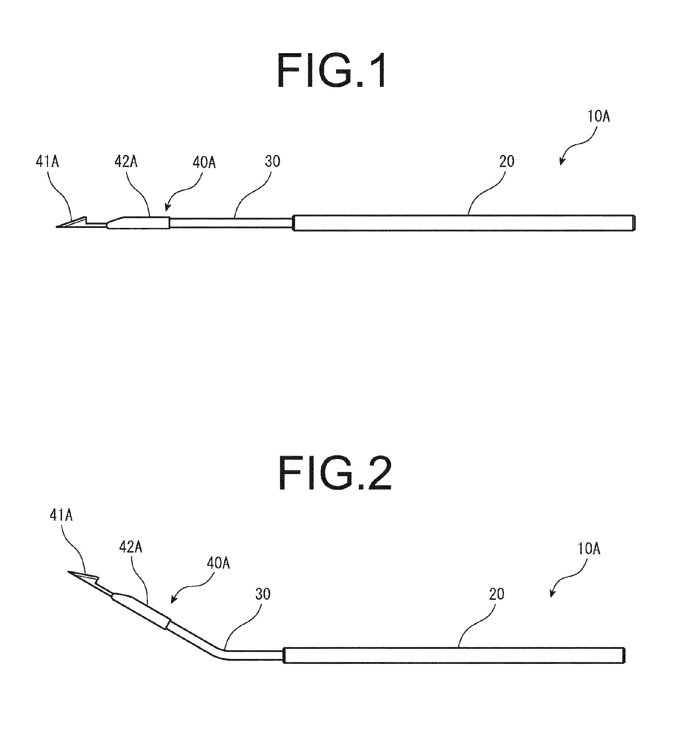

FIG. 1 is an entire front view showing the outline of a medical instrument according to Embodiment 1 of the invention. As shown in FIG. 1, a medical instrument (herein, medical knife) 10A of this Embodiment is comprised of a rod-shaped grasp portion 20, a shape memory portion 30 connected to the front end of the grasp portion 20, and a functional portion (herein, knife body) 40A disposed at the front end of the shape memory portion 30.

The grasp portion 20 is a portion that a user grasps in using, and is a rod-shaped member made of metal such as a titanium alloy and stainless steel. Knurling or pearskin finish is applied to the circumference of the grasp portion 20 in order for fingers not to slip when a user grasps and operates.

The shape memory portion 30 is a rod-shaped member, provided at the front end of the grasp portion 20, made of a shape memory alloy such as Nitinol (nickel-titanium alloy). The shape memory alloy is a specific alloy which memories some shape by heat treatment, and after deforming the alloy, returns to its shape before being deformed by heating the alloy to a certain temperature or more. The shape memory portion 30 is subjected to memory treatment in a straight line state as shown in FIG. 1. This shape memory portion 30 becomes deformed by force of the extent that is applied by an adult using both hands or a tool (pliers, etc.), but is provided with flexibility/rigidity of the extent that the portion 30 is not deformed during its use in surgery and the like.

The functional portion 40A is a knife body, herein, and is disposed at the front end of the shape memory portion 30. The knife body 40A is comprised of a blade body 41A provided at the front end, and a blade body holder 42A made of plastic or the like to hold the blade body 41A.

The grasp portion 20, shape memory portion 30 and functional portion 40A are connected to be mutually attachable and detachable. As a method of connecting the portions, it is possible to apply various schemes such as a fit scheme and screw scheme. Then, the grasp portion 20 and shape memory portion 30 are capable of being reused by sterilizing whenever they are used, and only the functional portion 40A is made disposable. Thus, as the functional portion 40A, a plurality of shape types is enclosed in individual storage means (not shown), and any one of the plurality of shape types is selected and installed in an installation portion provided at the front end of the shape memory portion to be used. Further, the functional portion enclosed inside the storage means is sterilized and stored.

In using the medical knife 10A of this Embodiment as described above, as shown in FIG. 2, an operator applies a force to the shape memory portion 30 as necessary using pliers or dedicated auxiliary tool (not shown), or manually to bend. Herein, as the dedicated auxiliary tool, in order to bend the shape memory portion 30 supporting the shape of each of the medical knife, medical mirror and medical hook with ease by a small force, for example, the tool using the principle of leverage is suitable. Further, the dedicated auxiliary tool may be provided with heating means (electric heat) to heat the shape memory portion 30 of the medical instrument 10A.

By this means, it is possible to provide the functional portion 40A with an arbitrary angle with respect to the grasp portion 20. In addition, herein, the shape memory portion 30 is bent to the blade side of the blade body 41A, but is capable of being bent in any free direction corresponding to a use and site to treat.

After using, the functional portion 40A is removed and discarded, and the grasp portion 20 and shape memory portion 30 are sterilized. Subsequently, the shape memory portion 30 is heated to a memory recovery temperature, and then, returns to its original shape i.e. the straight shape as shown in FIG. 1. It is suitable that the memory recovery temperature of the shape memory portion 30 is set at about 100°C, for example, and in this case, it is possible to recover the shape with ease by boiling or exposing to sterilization steam.

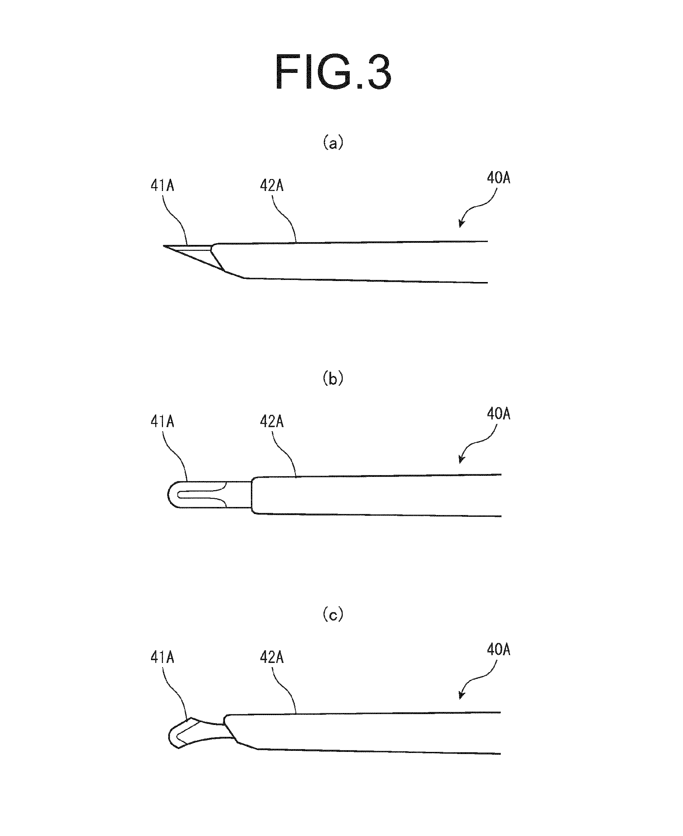

In addition, in the above-mentioned description, as the blade body 41A of the medical knife 10A, the blade body protruding substantially in the shape of a triangle is illustrated as an example, but it is possible to attach blade bodies of various shapes as well as such a shape. FIG. 3 shows example of blade bodies of various shapes. Then, the medical instrument 10 of the invention enables such a knife body 40A to be attached and detached freely.

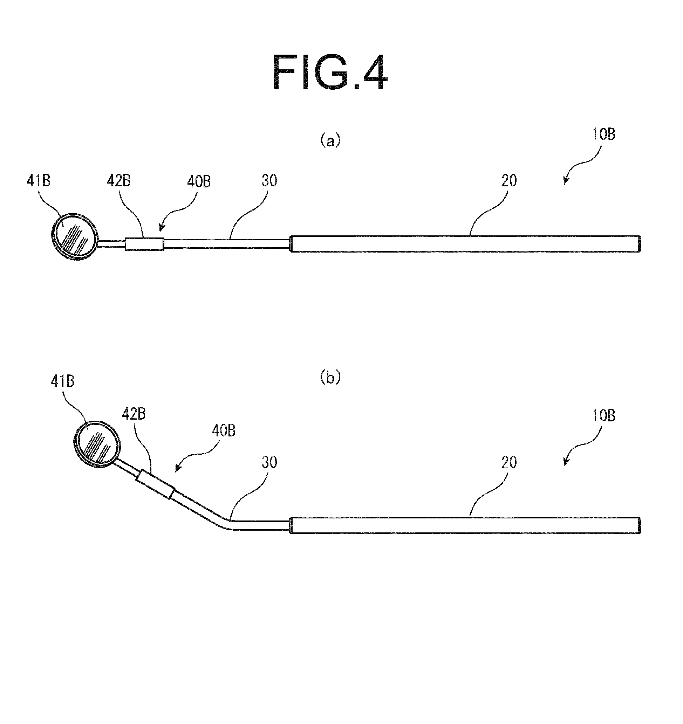

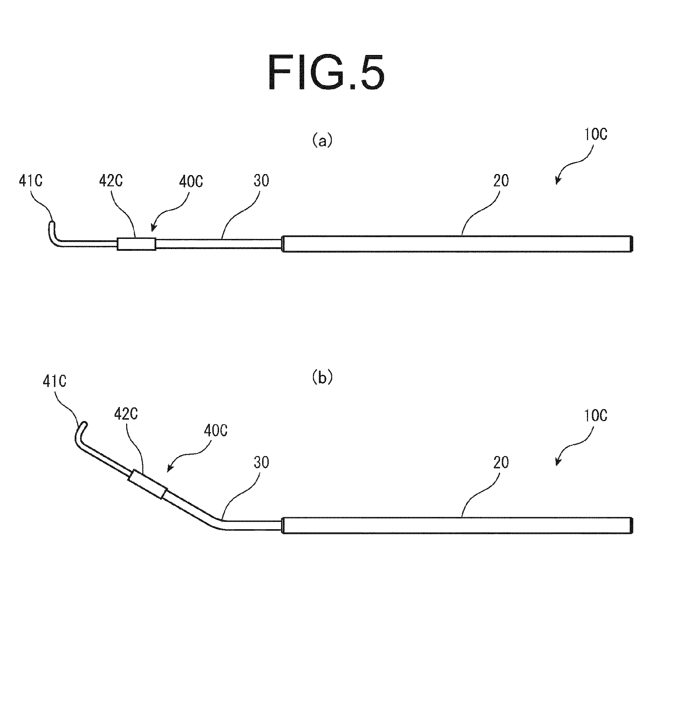

Further, in the above-mentioned description, the medical knife 10A is described as the medical instrument, but it is possible to apply various types to the functional portion as well as the knife. FIG. 4 shows a medical mirror 10B, and FIG. 5 shows a medical hook 10C. In addition, the same portions as in FIG. 1 are assigned the same reference numerals to omit specific descriptions.

First, as shown in FIG. 4, the medical mirror 10B is comprised of the rod-shaped grasp portion 20, the shape memory portion 30 connected to the front end of the grasp portion 20, and a functional portion (herein, mirror body) 40B disposed at the front end of the shape memory portion 30.

The mirror body 40B is comprised of a mirror 41B, and a mirror holder 42B that holds the mirror 41B. The mirror body 40B is connected to the shape memory portion 30 to be attachable and detachable, and it is possible to throw away only the mirror body 40B after using.

In using the medical mirror 10B, as shown in FIG. 4(b), the shape memory portion 30 is given a force as necessary and bent. By this means, it is possible to provide the functional portion 40B with an arbitrary angle with respect to the grasp portion 20.

After using, the functional portion 40B is removed and discarded, and the grasp portion 20 and shape memory portion 30 are sterilized. Subsequently, the shape memory portion 30 is heated to a memory recovery temperature, and then, returns to its original shape i.e. the straight shape as shown in FIG. 4(a).

Further, as shown in FIG. 5, the medical hook 10C is comprised of the rod-shaped grasp portion 20, the shape memory portion 30 connected to the front end of the grasp portion 20, and a functional portion (herein, hook body) 40C disposed at the front end of the shape memory portion 30.

The hook body 40C is comprised of a hook 41C, and a hook holder 42C that holds the hook 41C. The hook body 40C is connected to the shape memory portion 30 to be attachable and detachable, and it is possible to throw away only the hook body 40C after using.

In using the medical hook 10C, as shown in FIG. 5(b), the shape memory portion 30 is given a force as necessary and bent. By this means, it is possible to provide the functional portion 40C with an arbitrary angle with respect to the grasp portion 20.

After using, the functional portion 40C is removed and discarded, and the grasp portion 20 and shape memory portion 30 are sterilized. Subsequently, the shape memory portion 30 is heated to a memory recovery temperature, and then, returns to its original shape i.e. the straight shape as shown in FIG. 5(a).

As described above, in the medical instrument 10 of this Embodiment, each portion is made attachable/detachable, only the functional portion 40 is made disposable, consideration is thus given to safety and sterilization, while the cost is suppressed, and further, by providing the shape memory portion 30, it is possible to make the angle of the functional portion 40 adjustable freely, and to ensure excellent visual recognition.

### [Embodiment 2]

In addition, the shape memory alloy used in the shape memory portion in the invention is a specific alloy that returns to its original shape subjected to memory treatment by heating after being deformed, but when the alloy is given large deformation exceeding the limit, the alloy is not able to return to its original shape by heating. Therefore, it is necessary to consider not to deform the shape memory portion more than a certain range. Therefore, in the case of setting the functional portion for an angle exceeding the deformation limit of the shape memory portion such as the case of making the angle of the functional portion large with respect to the grasp portion, the medical instrument is configured as described below.

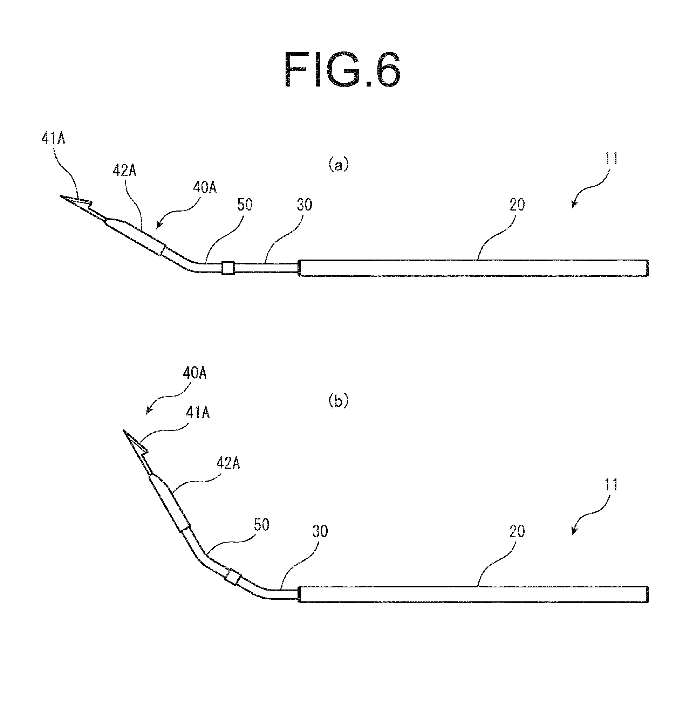

FIG. 6 contains entire front views showing the outline of a medical instrument according to Embodiment 2 of the invention. In addition, the same portions as in FIG. 1 are assigned the same reference numerals to omit specific descriptions.

As shown in FIG. 6, a medical instrument (herein, as an example, medical knife) 11 of this Embodiment is comprised of the rod-shaped grasp portion 20, the shape memory portion 30 connected to the front end of the grasp portion 20, a bent connection portion 50 disposed at the front end of the shape memory portion 30, and the functional portion (herein, knife body) 40A disposed at the front end of the shape memory portion 30. Thus, by arranging the connection portion 50 bent at a predetermined angle in between the grasp portion 20 and the functional portion 40A, it is possible to provide the functional portion 40A with a large angle without exceeding the deformation limit of the shape memory portion 30.

The connection portion 50 is made of a material such as stainless steel, and is beforehand bent at an arbitrary angle. It is preferable to prepare a plurality of connection portions 50 bent at various angles and use separately according to the case.

Further, the grasp portion 20, shape memory portion 30, connection portion 50 and functional portion 40A are connected to be mutually attachable and detachable. As a method of connecting the portions, it is possible to apply various schemes such as a fit scheme and screw scheme. Then, the grasp portion 20, shape memory portion 30 and connection portion 50 are capable of being reused by sterilizing whenever they are used, and only the functional portion 40A is made disposable.

In using the medical knife 11 of this Embodiment as described above, as shown in FIG. 6(b), the shape memory portion 30 is given a force and bent as necessary. The connection portion 50 is already bent, and therefore, it is possible to provide the functional portion 40A with a large angle without exceeding the deformation limit of the shape memory portion 30.

After using, the functional portion 40A is removed and discarded, and the grasp portion 20, shape memory portion 30 and connection portion 50 are sterilized. Subsequently, the shape memory portion 30 is heated to a memory recovery temperature, and then, returns to its original shape i.e. the straight shape as shown in FIG. 6(a). It is suitable that the memory recovery temperature of the shape memory portion 30 is set at about 100°C, for example, and in this case, it is possible to recover the shape with ease by boiling or exposing to sterilization steam.

In addition, shown herein is the example in which the connection portion 50 is arranged between the shape memory portion 30 and the functional portion 40, but the positions of the shape memory portion 30 and the connection portion 50 may be replaced reversely.

By configuring as described above, in the medical instrument 11 of this Embodiment, only the functional portion 40 is made disposable, consideration is thus given to safety and sterilization, while the cost is suppressed, and further, by providing the bent connection portion 50 together with the shape memory portion 30, it is possible to make the functional portion 40A adjustable freely to angles of the deformation limit or more of the shape memory portion 30 without exceeding the deformation limit of the shape memory portion 30, and to ensure excellent visual recognition.

In the above-mentioned descriptions, the Embodiments of the present invention are described, but the invention is not limited to the above-mentioned Embodiments, various modifications thereof can be made based on the subject matter of the invention, and the modifications are not excluded from the scope of the invention.

### Industrial Applicability

The present invention relates to medical instruments such as a knife, mirror and hook used in the medical field, and more particularly, to a medical instrument for enabling the functional portion provided at the front end of the instrument to be attached and detached, while enabling the angle between the functional portion and the grasp portion to be changed freely, and has industrial applicability. Description of Symbols

- 10A: Medical instrument (medical knife)
- 10B: Medical instrument (medical mirror)
- 10C: Medical instrument (medical hook)
- 11: Medical instrument (medical knife)
- 20: Grasp portion
- 30: Shape memory portion
- 40A: Functional portion (knife body)
- 40B: Functional portion (mirror body)
- 40C: Functional portion (hook body)
- 41A: Blade body
- 41B: Mirror
- 41C: Hook
- 42A: Blade body holder
- 42B: Mirror holder
- 42C: Hook holder
- 50: Connection portion

## Claims

1. A medical instrument provided with a rod-shaped grasp portion at a base end thereof and a functional portion used in a medical practice at a front end thereof,
wherein a shape memory portion made of a rod-shaped shape memory alloy is provided in between the grasp portion and the functional portion,
the grasp portion, the functional portion and the shape memory portion are made mutually attachable and detachable, and
the shape memory portion is subjected to memory treatment in a linear state, is bent as necessary to use, and is returned to an original shape thereof subjected to the memory treatment by heating the shape memory portion to a memory recovery temperature after using.

2. The medical instrument according to claim 1, wherein the instrument is further provided with a connection portion bent at a predetermined angle between the grasp portion and the shape memory portion or between the shape memory portion and the functional portion.

3. The medical instrument according to claim 1, wherein the shape memory portion is made of a nickel-titanium alloy.

4. The medical instrument according to claim 1 or 2, wherein the medical instrument is any one of a medical knife, a medical mirror, and a medical hook.

5. The medical instrument according to claim 4, wherein as the functional portion, a plurality of shape types is enclosed in individual storage means, and any one of the plurality of shape types is selected and installed in an installation portion of the shape memory portion to be used.

6. The medical instrument according to claim 5, wherein the functional portion is sterilized and stored in the storage means.
